# EUROPEAN PATENT APPLICATION

(11) **EP 2 889 018 A1**
(43) Date of publication of application: **01.07.2015**
(21) Application number: 12883840.6
(22) Date of filing: 27.08.2012
(51) Int. Cl.: A61F 2/08, A61B 17/00

(54) **MEDICAL TUBE AND MEDICAL TUBE ASSEMBLY**

(71) Applicant: Terumo Kabushiki Kaisha, Tokyo 151-0072 (JP)
(72) Inventor: KAWAURA, Masakatsu, Ashigarakamigun Kanagawa 259-0151 (JP); YOKOI, Nao, Ashigarakamigun Kanagawa 259-0151 (JP)
(74) Representative: Casalonga
(86) International application number: PCT/JP2012/071596
(87) International publication number: WO 2014/033814

(57) **Abstract**

Provided are a medical tube and a medical tube assembly which enable an implant to be easily and reliably inserted and placed to indwell in a living body. The medical tube 2 accepts an elongated implant 8 inserted therein. The medical tube 2 is composed of a tube which has a distal end opening 21 where its distal end is opening, and a proximal end opening 22 where its proximal end is opening. Besides, the tube has a curved section 231 where an intermediate portion in the longitudinal direction thereof is curved and where the curved state is maintained.

## Description

### Technical Field

The present invention relates to a medical tube and a medical tube assembly.

### Background Art

In a patent suffering from urinary incontinence, particularly stress urinary incontinence, urine leakage occurs due to an abdominal pressure exerted during a normal exercise or by laughing, coughing, sneezing or the like. This is attributable, for example, to loosening of the pelvic floor muscle, which is a muscle for supporting the urethra, caused by childbirth or the like.

For treatment of urinary incontinence, effective is surgical therapy, in which there is used, for example, a tape-shaped implant called "sling," and the sling is placed indwelling in the body, so as to support the urethra thereby (see, for example, Patent Document 1). In order to put a sling indwelling in the body, an operator incises the vagina with a surgical knife, exfoliates a biological tissue between the urethra and the vagina, and forms a puncture-through hole for providing communication between the exfoliated biological tissue and the exterior, by use of a puncture needle or the like. Then, the sling is inserted into the puncture-through hole, to be placed to indwell in the exfoliated biological tissue in the body.

Meanwhile, at the time of inserting the sling into the puncture-through hole, the inserting operation is conducted while the sling is kept inserted in a flexible tube. Since this tube is flexible, it would be crushed (compressed) by the exfoliated biological tissue. As a result, if the sling inserting operation is conducted, the sling-inserting operation may become difficult to carry out, due to the friction between the tube and the biological tissue.

### Prior Art Document

### Patent Document

Patent Document 1: Japanese Patent Laid-Open No. 2010-99499

### Summary of Invention

### Technical Problem

It is an object of the present invention to provide a medical tube and a medical tube assembly which ensure that an operation of inserting an implant into a living body so as to set the implant to indwell in the living body can be carried out easily and reliably.

### Technical Solution

The above object is achieved by the present invention as described in the following paragraphs (1) to (15).
(1) A medical tube in which an elongated implant is to be inserted, including
   a tube having a distal end opening where a distal end thereof is opening, and a proximal end opening where a proximal end thereof is opening, the tube having a curved section where an intermediate portion in a longitudinal direction thereof is curved and where a curved state is maintained.
(2) The medical tube as described in the above paragraph (1), wherein the tube is rigid at least at the curved section thereof.
(3) The medical tube as described in the above paragraph (1) or (2), wherein the curved section is curved in a circular arc shape.
(4) The medical tube as described in any of the above paragraphs (1) to (3), including
   a separation part where the tube is separable at an intermediate portion in the longitudinal direction thereof.
(5) The medical tube as described in the above paragraph (4), wherein the separation part is disposed at a central portion in a longitudinal direction of the curved section.
(6) The medical tube as described in the above paragraph (5), wherein the tube is provided with a marker for grasping of the central portion in the longitudinal direction of the curved section.
(7) The medical tube as described in any of the above paragraphs (4) to (6),
   wherein the tube is separated at the separation part into a first tube on a distal side and a second tube on a proximal side; and
   the separation part is a part having a fitting structure in which a distal portion of the second tube is fitted in a proximal portion of the first tube, before separation of the tube into the first tube and the second tube.
(8) The medical tube as described in any of the above paragraphs (1) to (7), wherein the medical tube is circular in cross-sectional shape at least near the distal end opening.
(9) The medical tube as described in the above paragraph (8), wherein an enlarged diameter portion where the medical tube is enlarged in outside diameter is provided near the distal end opening.
(10) The medical tube as described in the above paragraph (8) or (9), wherein the medical tube is flat in cross-sectional shape at its portion proximally of its portion near the distal end opening.
(11) The medical tube as described in the above paragraph (10), wherein the width of the flat shape is equal to or greater than a maximum outside diameter near the distal end opening of the medical tube.
(12) The medical tube as described in the above paragraph (10) or (11), wherein a thickness direction of the flat shape is oriented toward a center-of-curvature of the curved section.
(13) The medical tube as described in any of the above paragraphs (1) to (12), including
   at least one lumen which opens respectively at the distal end opening and at the proximal end opening,
   wherein the implant is inserted in the lumen.
(14) The medical tube as described in the above paragraph (13), including
   a plurality of the lumens,
   wherein a guide wire is inserted in each of other lumen or lumens than that one of the lumens in which the implant is inserted.
(15) A medical tube assembly including:
   the medical tube as described in any of the above paragraphs (1) to (14); and
   an elongated implant which is inserted in the medical tube.

### Advantageous Effect

According to the present invention, at the time of inserting the medical tube into a living body, the curved section is prevented from being crushed (compressed) inside the living body. This ensures that, for example, in the case where an implant is preliminarily inserted in the medical tube, an operation of inserting the medical tube into a living body together with the implant can be carried out easily and assuredly.

In addition, after the inserting operation, it is required only to pull the medical tube out of the living body, whereby the implant is left as it is, so that the implant is easily and reliably placed to indwell in the living body.

Besides, where the medical tube has the separation part, the medical tube can be separated at the separation part. This makes it possible to easily carry out an operation of drawing the medical tube out of a living body, and, therefore, to speedily place an implant indwelling in the living body.

### Brief Description of Drawings

[FIG. 1]

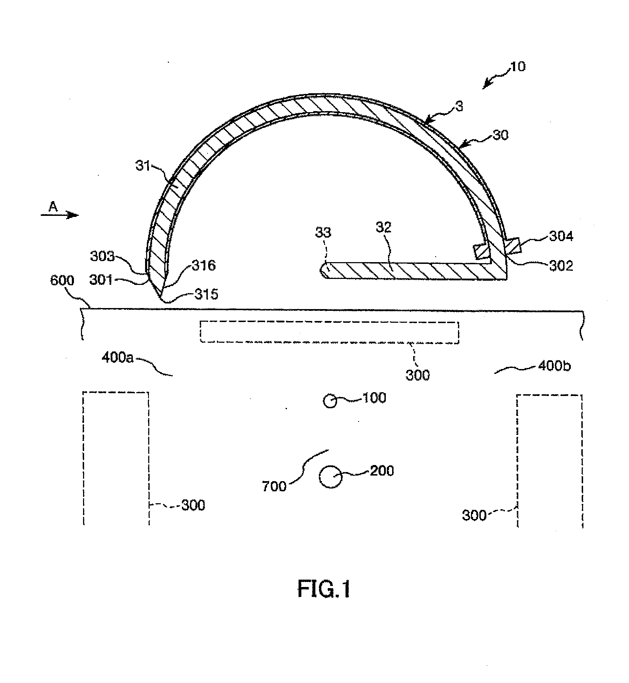

   FIG. 1 is a sectional view for sequentially illustrating a method of using the medical tube (medical tube assembly) of the present invention in a first embodiment thereof.
[FIG. 2]

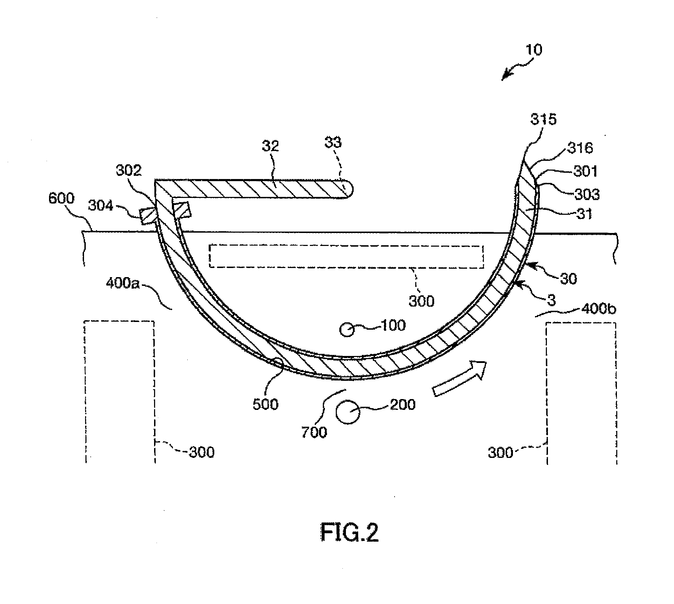

   FIG. 2 is a sectional view for sequentially illustrating the method of using the medical tube (medical tube assembly) of the present invention in the first embodiment thereof.
[FIG. 3]

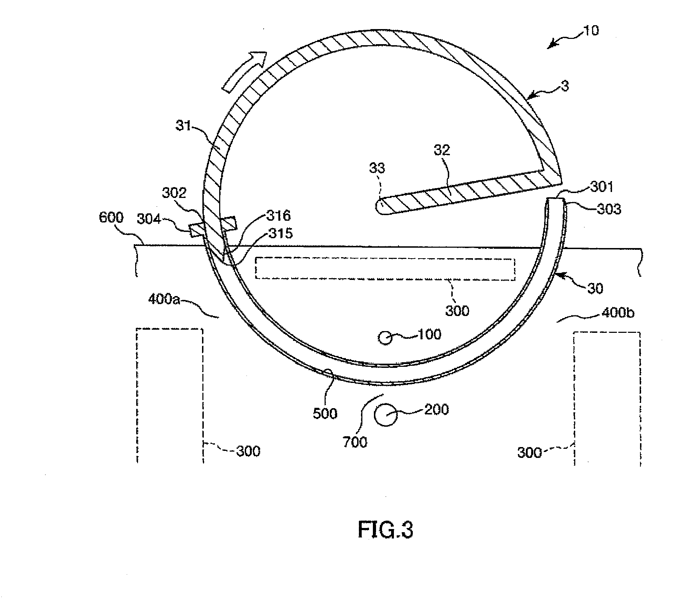

   FIG. 3 is a sectional view for sequentially illustrating the method of using the medical tube (medical tube assembly) of the present invention in the first embodiment thereof.
[FIG. 4]

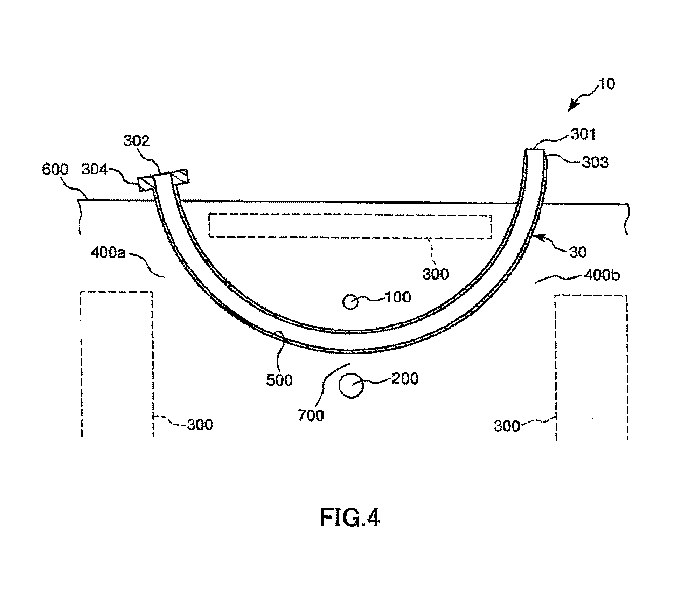

   FIG. 4 is a sectional view for sequentially illustrating the method of using the medical tube (medical tube assembly) of the present invention in the first embodiment thereof.
[FIG. 5]

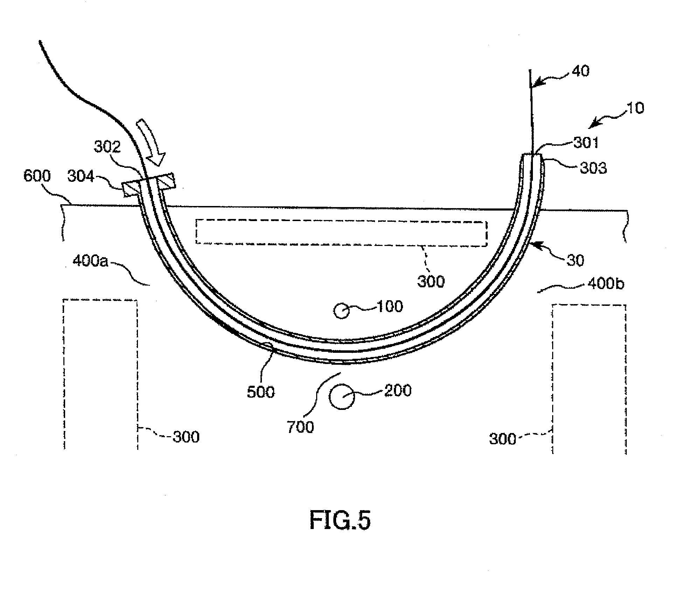

   FIG. 5 is a sectional view for sequentially illustrating the method of using the medical tube (medical tube assembly) of the present invention in the first embodiment thereof.
[FIG. 6]

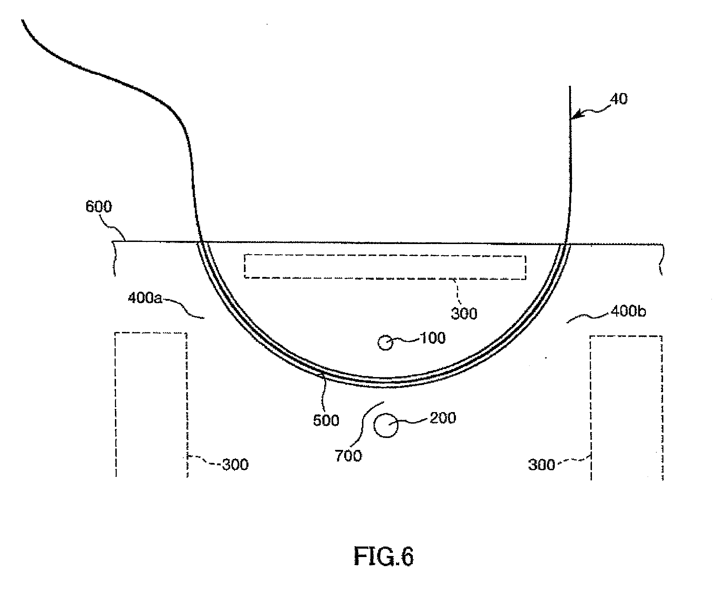

   FIG. 6 is a sectional view for sequentially illustrating the method of using the medical tube (medical tube assembly) of the present invention in the first embodiment thereof.
[FIG. 7]

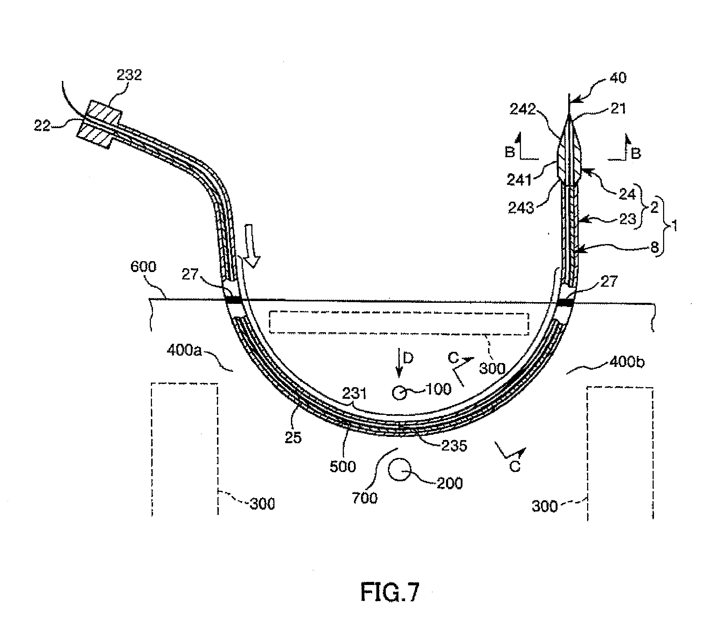

   FIG. 7 is a sectional view for sequentially illustrating the method of using the medical tube (medical tube assembly) of the present invention in the first embodiment thereof.
[FIG. 8]

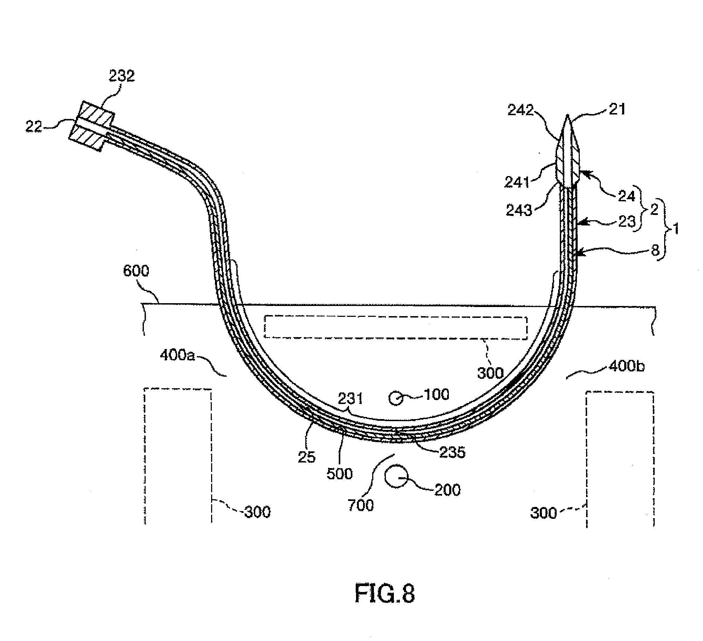

   FIG. 8 is a sectional view for sequentially illustrating the method of using the medical tube (medical tube assembly) of the present invention in the first embodiment thereof.
[FIG. 9]

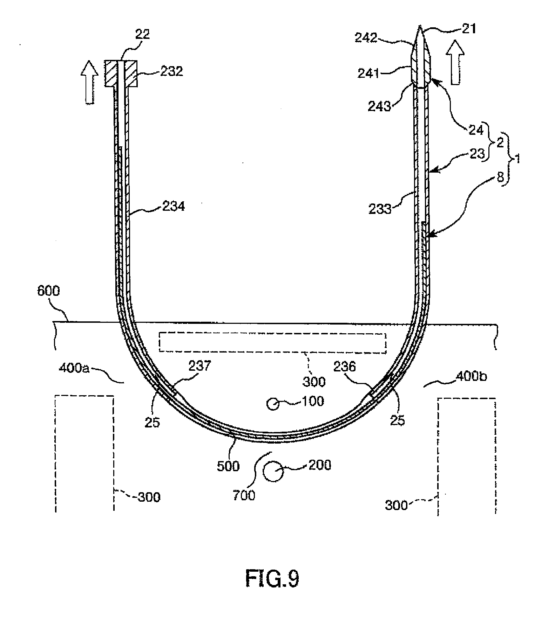

   FIG. 9 is a sectional view for sequentially illustrating the method of using the medical tube (medical tube assembly) of the present invention in the first embodiment thereof.
[FIG. 10]

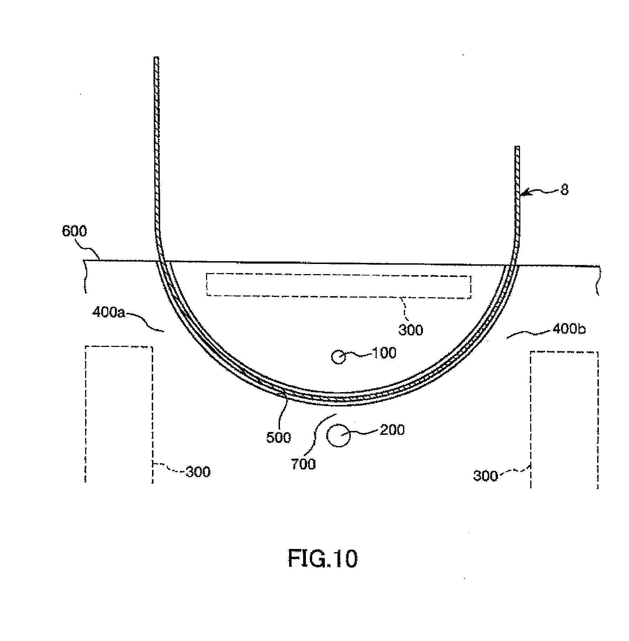

   FIG. 10 is a sectional view for sequentially illustrating the method of using the medical tube (medical tube assembly) of the present invention in the first embodiment thereof.
[FIG. 11]

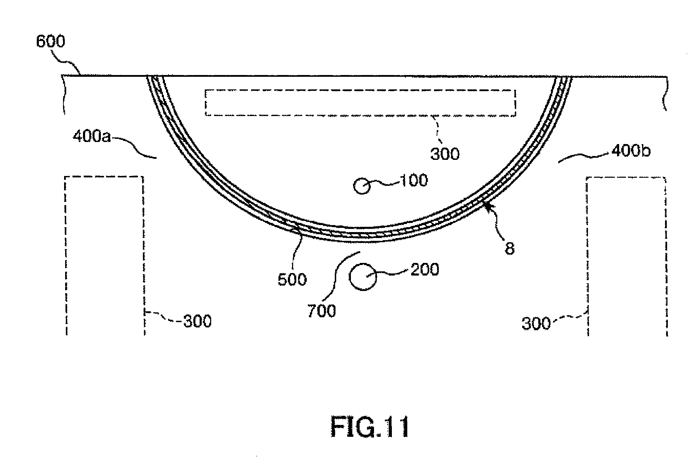

   FIG. 11 is a sectional view for sequentially illustrating the method of using the medical tube (medical tube assembly) of the present invention in the first embodiment thereof.
[FIG. 12]

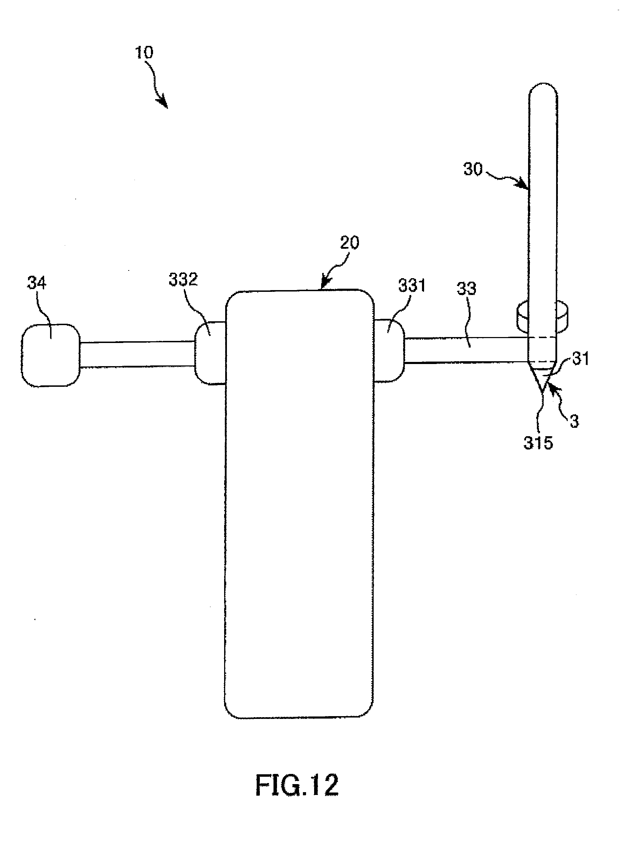

   FIG. 12 is a view (side view) taken in the direction of arrow A in FIG. 1.
[FIG. 13]

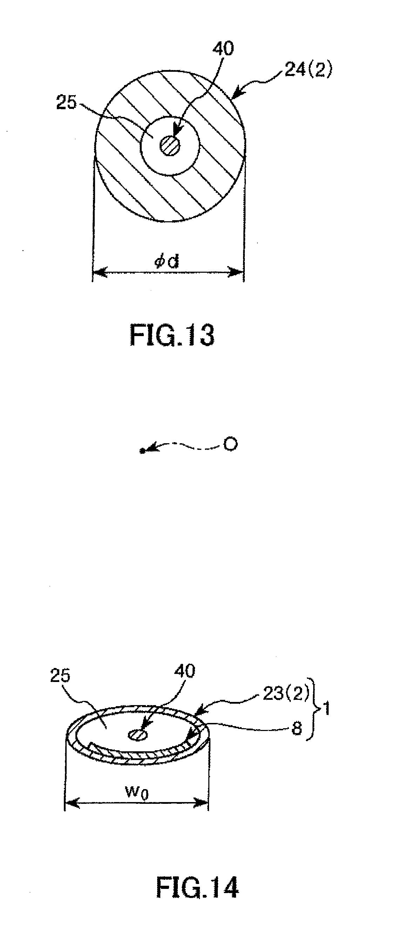

   FIG. 13 is a sectional view taken along line B-B in FIG. 7.
[FIG. 14]
   FIG. 14 is a sectional view taken along line C-C in FIG. 7.
[FIG. 15]

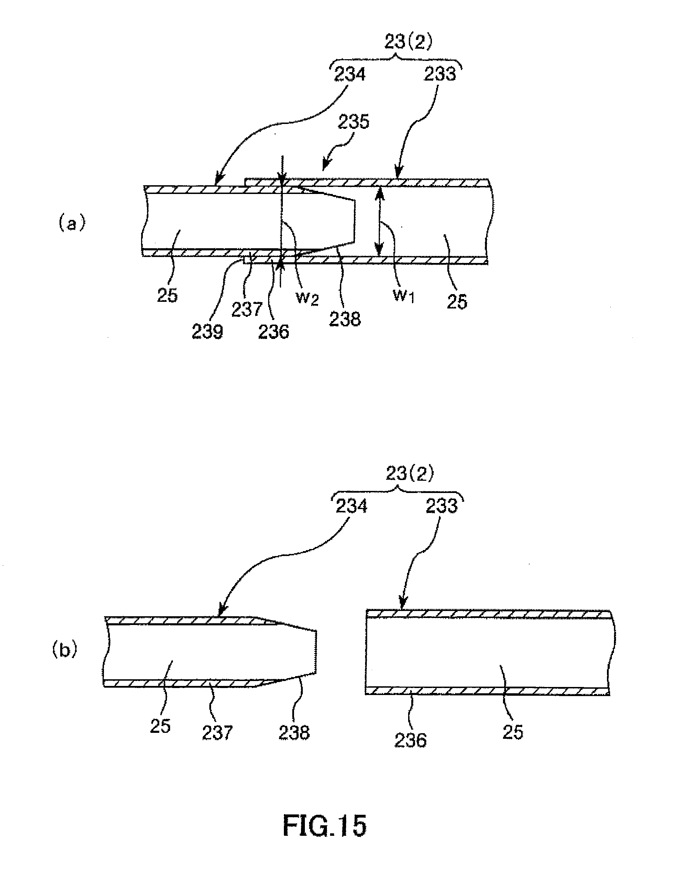

   FIG. 15 shows views taken in the direction of arrow D in FIG. 7 (wherein (a) shows a state before separation of a separation part, and (b) shows a state after separation of the separation part).
[FIG. 16]

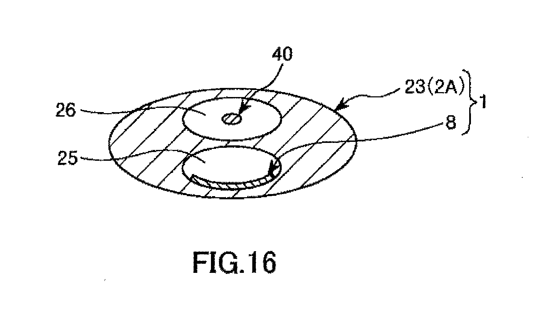

   FIG. 16 is a cross-sectional view showing a second embodiment of the medical tube (medical tube assembly) of the present invention.
[FIG. 17]

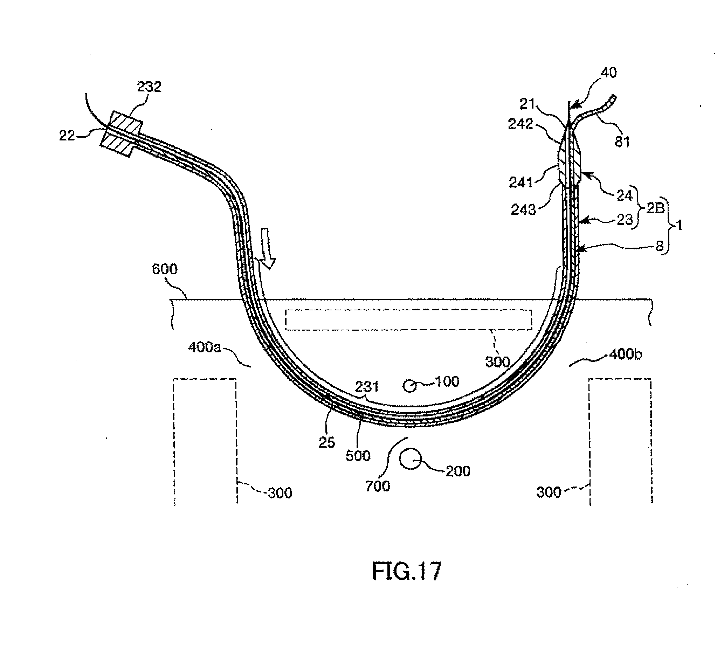

   FIG. 17 is a sectional view for sequentially illustrating a method of using the medical tube (medical tube assembly) of the present invention in a third embodiment thereof.
[FIG. 18]

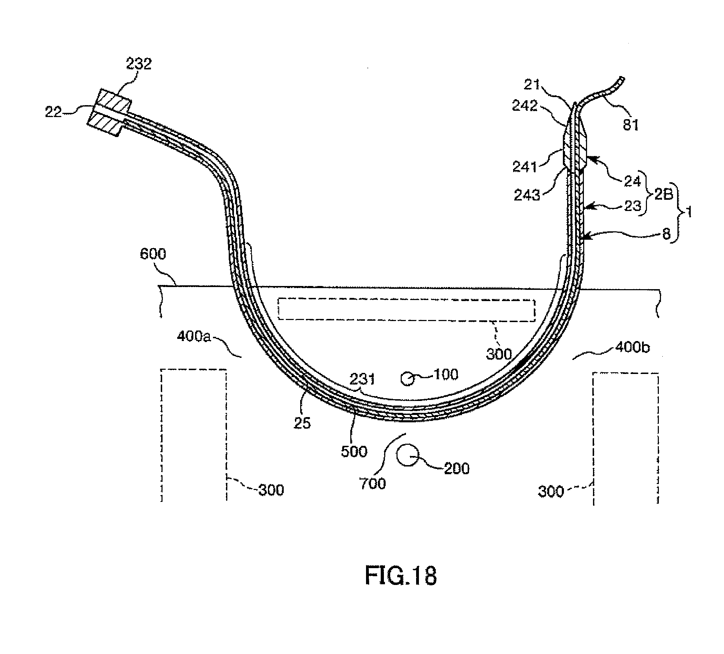

   FIG. 18 is a sectional view for sequentially illustrating the method of using the medical tube (medical tube assembly) of the present invention in the third embodiment thereof.
[FIG. 19]

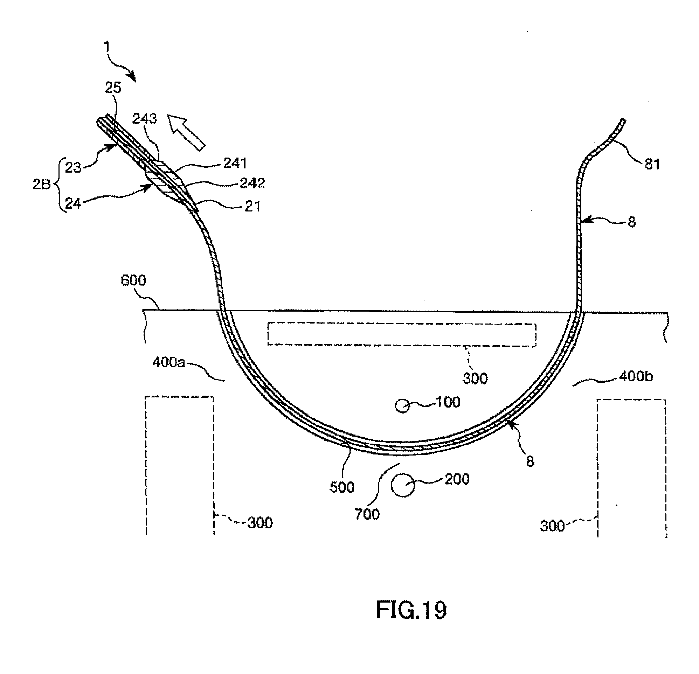

   FIG. 19 is a sectional view for sequentially illustrating the method of using the medical tube (medical tube assembly) of the present invention in the third embodiment thereof.

### Modes for Carrying Out the Invention

Hereafter, the medical tube and the medical tube assembly of the present invention will be described in detail based on preferred embodiments thereof shown in the attached drawings.

### <First Embodiment>

FIGS. 1 to 11 are sectional views for sequentially illustrating a method of using the medical tube (medical tube assembly) of the present invention in a first embodiment thereof; FIG. 12 is a view (side view) taken in the direction of arrow A in FIG. 1; FIG. 13 is a sectional view taken along line B-B in FIG. 7; FIG. 14 is a sectional view taken along line C-C in FIG. 7; and FIG. 15 shows views taken along the direction of arrow D in FIG. 7 (wherein (a) shows a state before separation of a separation part, and (b) shows a state after separation of the separation part). Note that hereafter, for convenience of description, the upper side in FIGS. 1 to 12 (and in FIGS. 17 to 19, as well) will be referred to as "upper (side)" or "above," and the lower side as "lower (side)" or "below." In addition, the side of a needle tip will be referred to as "distal end," and the opposite side as "proximal end."

A medical tube assembly 1 shown in FIGS. 7 and 8 includes a medical tube (tube) 2, and an implant 8 which is inserted in the medical tube 2. This medical tube assembly 1 is a medical instrument to be used for treatment of female urinary incontinence. Now, the configurations of components will be described.

First, the implant 8 will be described.

The implant 8, which is generally called "sling," is an implantable instrument for treatment of female urinary incontinence, specifically, an instrument for supporting a urethra 100, more specifically, an instrument which supports the urethra 100 in the manner of pulling the urethra 100 in a direction for spacing away from a vagina 200 when the urethra 100 would tend to move toward the vagina 200 side, for example (see FIG. 11). The implant 8 is composed of a member which is flexible and band-like (elongated) in shape (see FIGS. 7 to 11).

The material constituting the implant 8 is not specifically restricted; for example, various resin materials that are biocompatible can be used as the material.

Note that the implant 8 may be preliminarily inserted (housed) in the medical tube 2 as illustrated in FIG. 8, or may be inserted into the medical tube 2 in the course of a procedure. Where the implant 8 is preliminarily inserted in the medical tube 2, a speedy procedure can be performed. Where the implant 8 is inserted into the medical tube 2 in the course of a procedure, an implant 8 suited to the individual case can be selected each time, according to the case. In this embodiment, a case where the implant 8 is preliminarily inserted in the medical tube 2 will be described on a representative basis.

In the next place, prior to describing the medical tube 2, a puncture apparatus 10 to be used together with the medical tube assembly 1 in treatment of female urinary incontinence will be described. Note that it can be said that, in this embodiment, a "medical instrument set" for treatment of female urinary incontinence is composed of the puncture apparatus 10 and the medical tube assembly 1.

As shown in FIGS. 1 to 5 and 12, the puncture apparatus 10 includes a puncture member 3, a support member 20 supporting the puncture member 3 in a rotatable manner, and an outer tube 30 in which to insert the puncture member 3. Note that the puncture apparatus 10 may further include a bar-shaped urethral-insertion member to be inserted into the urethra 100, and a bar-shaped vaginal-insertion member to be inserted into the vagina 200. These members are each preferably supported by and fixed to the support member 20.

The puncture member 3 includes a puncture needle 31 for puncturing a biological tissue 700, a shaft portion 33, and an interlock portion 32 that interlocks the puncture needle 31 and the shaft portion 33.

The puncture needle 31 has a sharp needle tip 315 at the distal end thereof, and is curved in a circular arc shape with a center on the shaft portion 33. In addition, the axis of the puncture needle 31 and the axis of the shaft portion 33 are in a skew-lines relationship. This ensures that when the puncture member 3 is rotationally moved about the shaft portion 33, the needle tip 315 of the puncture needle 31 is moved along the circular arc, in a plane orthogonal to the axis of the shaft portion 33, namely, in a plane such that the axis of the shaft portion 33 constitutes a normal to the plane.

Note that the center angle of the circular arc of the puncture needle 31 is not particularly limited but is set, as required, according to various conditions. In this case, the center angle is so set that when the biological tissue 700 is punctured by the puncture needle 31, a puncture-through hole (puncture hole) 500 having a circular arc shape is formed in the biological tissue 700, as will be described later. Such a center angle is, for example, preferably 120 to 270 degrees, more preferably 160 to 230 degrees, and further preferably 180 to 210 degrees.

In addition, while the needle tip 315 of the puncture needle 31 is oriented counterclockwise in FIGS. 1 to 3 in this embodiment, this is not restrictive, and the needle tip 315 may be oriented clockwise.

Besides, the puncture needle 31 is formed with a tapered portion 316 where its outside diameter gradually increases along the proximal direction from the needle tip 315.

In addition, the puncture needle 31 may be either a solid needle or a hollow needle.

The shaft portion 33 serves as a rotating shaft of the puncture member 3 (puncture needle 31), and is rotatably disposed on the support member 20.

As shown in FIG. 12, the shaft portion 33 penetrates the support member 20 in the left-right direction in the figure. Besides, a flange 331 and a flange 332 are formed respectively at a distal-side portion and a proximal-side portion of the shaft portion 33, with the support member 20 interposed therebetween. The flanges 331 and 332 restrict movement of the shaft portion 33 in the axial direction relative to the support member 20.

In addition, at an end portion of the shaft portion 33 on the side opposite to the puncture needle 31, there is provided a grasping unit 34 as an operation unit for rotationally operating the puncture member 3. The grasping unit 34 is in the shape of a rectangular parallelepiped in this embodiment. At the time of rotationally moving the puncture member 3, the grasping unit 34 is grasped with fingers and is rotated in a predetermined direction. Note that the shape of the grasping unit 34 is naturally not restricted to the just-mentioned shape.

The interlock portion 32 is a portion that interlocks the proximal end of the puncture needle 31 and the shaft portion 33.

The material constituting the puncture member 3 is not specifically restricted; for example, various metallic materials such as stainless steels, aluminum or aluminum alloys, titanium or titanium alloys, etc. can be used as the material.

The support member 20 is a member that supports the puncture member 3 in a rotatable manner. Note that the support member 20 is omitted in FIGS. 1 to 3.

The support member 20 restricts the position of the puncture member 3 in such a manner that the needle tip 315 of the puncture needle 31 passes between the urethra 100 and the vagina 200 when the puncture member 3 is rotationally moved to puncture the biological tissue 700. This ensures that the puncture-through hole 500 having a circular arc shape is formed by the puncture needle 31, between the urethra 100 and the vagina 200.

The material constituting the support member 20 is not particularly limited; for example, various resin materials such as polyethylene, polypropylene, etc. can be used as the material.

As shown in FIGS. 1 and 2, the outer tube 30 is a member which is preliminarily mounted to the puncture needle 31 of the puncture member 3, that is, a member in which the puncture needle 31 of the puncture member 3 is preliminarily inserted. Note that the outer tube 30 is preferably a rigid tube. In this case, the outer tube 30 is curved in a circular arc shape, to the same extent as the puncture needle 31. Here, the term "rigid" refers to a degree of rigidity such that the outer tube 30 can, by itself, maintain the curved state in the circular arc shape.

The outer tube 30 has a distal end opening 301 where its distal end is opening, and a proximal end opening 302 where its proximal end is opening.

In addition, a distal portion of the outer tube 30 is formed at its outer circumferential portion with a tapered portion 303 which is equal in taper angle to the tapered portion 316 of the puncture needle 31. In an assembled state wherein the outer tube 30 is mounted to the puncture needle 31 to achieve assembly, the tapered portion 316 of the puncture needle 31 and the tapered portion 303 of the outer tube 30 constitute a single continuous tapered section. This ensures that in the assembled state, the outer tube 30 is, together with the puncture needle 31, able to puncture the biological tissue 700 and, therefore, to form the puncture-through hole 500 in the biological tissue 700 (see FIG. 2).

Besides, a proximal portion of the outer tube 30 is formed at its outer circumferential portion with a flange portion 304 where its outside diameter is enlarged. In the case where the flange portion 304 is attached to a living body surface 600 in a state as shown in FIG. 2, for example, a limit on rotary movement in the distal direction of the puncture needle 31 is restricted, so that the biological tissue 700 can be punctured neither too much nor too little.

The material constituting the outer tube 30 is not specifically restricted; for example, various resin materials such as polyethylene, polypropylene, etc. and various metallic materials such as stainless steels, aluminum or aluminum alloys, titanium or titanium alloys, etc. can be used as the material.

Now, the medical tube 2 will be described below.

As shown in FIGS. 7 to 9, the medical tube 2 is a tube which has a distal end opening 21 where its distal end is opening, and a proximal end opening 22 where its proximal end is opening. In addition, the medical tube 2 can be sectioned into an elongated tube main body 23, and a head part 24 provided at a distal portion of the tube main body 23. Besides, the medical tube 2 is formed therein with a lumen 25 which penetrates the tube main body 23 and the head part 24, in other words, which opens respectively at the distal end opening 21 and at the proximal end opening 22. In the lumen 25, there can be inserted the implant 8.

As shown in FIGS. 7 and 8, the tube main body 23 has a curved section 231 where an intermediate portion in the longitudinal direction thereof is curved in a circular arc shape. Of the medical tube 2, at least the curved section 231 is rigid. Here, the term "rigid" refers to a degree of rigidity such that the curved section 231 can, by itself, maintain the curved state in the circular arc shape. In addition, the degree of curling (curvature) of the curved section 231 is substantially the same as that of the puncture needle 31 of the puncture apparatus 10.

Such a configuration ensures that when the medical tube 2 is inserted into the puncture-through hole 500 formed by the puncture apparatus 10, the curved section 231 is prevented from being crushed (compressed) within the puncture-through hole 500, and the curved section 231 can easily be shaped following (along) the curved shape of the puncture-through hole 500. This enables the operation of inserting the medical tube 2 into the puncture-through hole 500 (living body) together with the implant 8 to be carried out easily and reliably. Besides, by separating the medical tube 2 as will be described later, after this inserting operation, the implant 8 can easily and assuredly be placed to indwell in the puncture-through hole 500 (see FIG. 9).

Note that, of the tube main body 23, the other portion than the curved section 231 may be rigid or may be non-rigid, namely, flexible.

As shown in FIG. 14, the cross-sectional shape of the tube main body 23, namely, the cross-sectional shape of that portion of the medical tube 2 which is on the proximal side of the head part 24 (the vicinity of the distal end opening 21), is a flat shape, specifically, an elliptic shape. This ensures that at the time of inserting the band-shaped implant 8 into the lumen 25 preliminarily, the inserting operation can be carried out easily. In addition, there is a merit that a space allowing reliable insertion of the implant 8 can be formed inside the puncture-through hole 500, and, further, the orientation of the implant 8 can be restricted.

Note that the cross-sectional shape of the tube main body 23 may be other than the flat shape; for example, a circular shape is applicable.

Besides, as shown in FIG. 14, the thickness direction (minor-diameter direction) of the flat shape is oriented toward the center-of-curvature O of the curved section 231. This configuration contributes to easier insertion of the medical tube 2 into the puncture-through hole 500, as compared with the case where the width direction (major-diameter direction) of the flat shape is oriented toward the center-of-curvature O.

As shown in FIGS. 7 to 9, a proximal portion of the tube main body 23 is formed with a flange portion 232 where its outside diameter is enlarged. Note that this flange portion 232 may be omitted.

As shown in FIGS. 9 and 15(b), the tube main body 23 (medical tube 2) is so configured that the tube main body 23 can be separated at an intermediate part in the longitudinal direction thereof, and that the tube main body 23 is thereby separated into a first tube 233 on the distal side and a second tube 234 on the proximal side. This separation makes it possible to swiftly draw the medical tube 2 out of the puncture-through hole 500, and, therefore, to put only the implant 8 indwelling in the puncture-through hole 500.

As shown in FIGS. 7 and 8, this separation part 235 is disposed at a central portion in the longitudinal direction of the curved section 231. This ensures that when the tube main body 23 is separated at the separation part 235, as shown in FIG. 9, the urethra 100 can be suitably supported by the implant 8.

The tube main body 23 is preferably provided, respectively on its distal portion and its proximal portion, with markers 27 for grasping of the central portion in the longitudinal direction of the curved section 231 (see FIG. 7). The markers 27 enable reliable grasping of the position of the central portion in the longitudinal direction of the curved section 231, in other words, the position of the separation part 235. Note that while the markers 27 are provided on both the distal portion and the proximal portion of the tube main body 23 in this embodiment, this configuration is not restrictive; for example, a marker may be provided on one of the distal portion and the proximal portion of the tube main body 23.

As shown in FIG. 15(a), the separation part 235 is a part having a fitting structure wherein a distal portion 237 of the second tube 234 is fitted inside a proximal portion 236 of the first tube 233, before separation of the tube main body 23 into the first tube 233 and the second tube 234. This ensures that by pulling the tube main body 23 toward both sides thereof, the fitted state of the proximal portion 236 of the first tube 233 and the distal portion 237 of the second tube 234 can be canceled assuredly. By this canceling, the tube main body 23 can be easily separated into the first tube 233 and the second tube 234 at the separation part 235.

Note that the distal portion 237 of the second tube 234 is formed with a gradually decreasing width portion 238 where its width decreases gradually along the distal direction. This ensures that at the time of keeping the tube main body 23 in the state shown in FIG. 15(a), the distal portion 237 of the second tube 234 can be easily inserted into the proximal portion 236 of the first tube 233, resulting in the fitted state of the end portions of them.

In addition, the inside width w₁ of the proximal portion 236 of the first tube 233 is equal to or slightly smaller than the outside width (maximum width) w₂ of the distal portion 237 of the second tube 234. This enables the distal portion 237 of the second tube 234 to be easily inserted into the proximal portion 236 of the first tube 233.

As aforementioned, the fitting structure between the proximal portion 236 of the first tube 233 and the distal portion 237 of the second tube 234 is in a state wherein the proximal portion 236 is located outside whereas the distal portion 237 is located inside. In this case, at the separation part 235, specifically at a boundary part between the proximal portion 236 and the distal portion 237, there is formed a stepped part 239 where the width is reduced stepwise along the proximal direction (see FIG. 15(a)). The configuration in which the stepped part 239 is formed in this way is preferable, since the medical tube 2 is inserted into the puncture-through hole 500, its distal end first.

As shown in FIGS. 7 to 9 and 13, the head part 24 is composed of a tubular body which is circular in cross-sectional shape. Note that the head part 24 may be formed integrally with the tube main body 23, or may be composed of a separate body from the tube main body 23 and be joined to the tube main body 23.

The head part 24 is provided, at an intermediate portion in the axial direction thereof, with an enlarged diameter portion 241 where its outside diameter is enlarged. In addition, that portion of the head part 24 which is on the distal side of the enlarged diameter portion 241 is a tapered portion 242 where its outside diameter gradually decreases along the distal direction, and that portion of the head part 241 which is on the proximal side of the enlarged diameter portion 241 is also a tapered portion 243 where its outside diameter gradually decreases along the proximal direction. Note that the whole length of the tapered portion 242 is longer than the whole length of the tapered portion 243.

With the head part 24 shaped in this fashion, the medical tube 2 can be easily inserted into the puncture-through hole 500, starting with its head part 24.

In addition, as shown in FIGS. 13 and 14, the outside width w₀ of the tube main body 23, which is flat shaped in cross section, is equal to or greater than the outside diameter ϕd of the head part 24, which is circular in cross-sectional shape. This ensures that as the medical tube 2 is inserted into the puncture-through hole 500, the puncture-through hole 500 can be dilated by the tube main body 23, in other words, the biological tissue 700 can be exfoliated.

Note that the material constituting the medical tube 2 is not specifically restricted, and examples of the applicable material include polyolefins such as polyethylene, polypropylene, ethylene-vinyl acetate copolymer, etc., modified polyolefins, polyamides (e.g., nylon 6, nylon 46, nylon 66, nylon 610, nylon 612, nylon 11, nylon 12, nylon 6-12, nylon 6-66), thermoplastic polyimides, liquid crystal polymers such as aromatic polyesters, etc., polyphenylene oxide, polyphenylene sulfide, polycarbonate, polymethyl methacrylate, polyethers, polyether-ether ketone, polyether imides, polyacetal, various thermoplastic elastomers based on styrene, polyolefin, polyvinyl chloride, polyurethane, polyester, polyamide, polybutadiene, transpolyisoprene, fluoro-rubber, chlorinated polyethylene or the like, and copolymers, blends, polymer alloys and the like that contain these elastomers as main constituents; these materials can be used either singly or as a mixture of two or more of them.

Now, an example of the method of using the medical tube assembly 1 (medical instrument set) will be described below, referring to FIGS. 1 to 11.
[1] First, as shown in FIG. 1, the puncture apparatus 10 in the assembled state in which the outer tube 30 is mounted to the puncture needle 31 to achieve assembly is mounted to the body surface 600 of a patient. The mounting position in this instance may be a position suitable for supporting the urethra 100 by the implant 8 which is to be implanted.
[2] Next, the grasping unit 34 of the puncture apparatus 10 is grasped with one hand, and, as shown in FIG. 2, the puncture member 3 is rotated counterclockwise in the figure. As a result, the puncture needle 31, together with the outer tube 30, is moved with the shaft portion 33 as a center of rotary movement to progressively pass, or puncture, a left-side inguinal region (or a part in the vicinity thereof) of the body surface 600 of the patient, an obturator foramen 400a of a pelvis 300, a region between the urethra 100 and the vagina 200, an obturator foramen 400b of the pelvis 300, and a right-side inguinal region (or a part in the vicinity thereof) of the body surface 600. By this puncture, the puncture-through hole 500 is formed to penetrate the biological tissue 700, extending from the left-side inguinal region to the right-side inguinal region of the body surface 600.
[3] Subsequently, the grasping unit 34 of the puncture apparatus 10 is grasped with one hand, the flange portion 304 of the outer tube 30 is pressed from above by the other hand, and, as shown in FIG. 3, the puncture member 3 is rotated in a direction opposite to the aforementioned, namely, rotated clockwise in the figure. As a result, the puncture needle 31 of the puncture member 3 is drawn out of the outer tube 30. Besides, the outer tube 30 is left indwelling in the biological tissue 700.
[4] Next, as shown in FIG. 4, the puncture member 3 of the puncture apparatus 10 is removed from the body surface 600, together with the support member 20.
[5] Subsequently, as shown in FIG. 5, a guide wire 40 is inserted into and passed through the outer tube 30 kept indwelling in the biological tissue 700. This results in that the guide wire 40 has its distal portion protruding from the distal end opening 301 of the outer tube 30, and has its proximal portion protruding from the proximal end opening 302 of the outer tube 30.
[6] Next, a distal portion of the guide wire 40 is grasped with one hand, and, keeping this condition, the flange portion 304 of the outer tube 30 is grasped with the other hand and pulled proximally. Consequently, as shown in FIG. 6, the outer tube 30 is drawn out of the puncture-through hole 500, whereas the guide wire 40 is left extending through the puncture-through hole 500.
[7] Subsequently, the medical tube assembly 1 in a state where the implant 8 is inserted in the medical tube 2 is prepared. Then, in a state where a proximal portion of the guide wire 40 is inserted in the distal end opening 21 of the medical tube 2, the medical tube assembly 1 is pushed distally relative to the guide wire 40, as shown in FIG. 7. This causes the medical tube assembly 1 to be inserted into and passed through the puncture-through hole 500, and brought into a state in which its distal-side portion is protruding from the body surface 600 on the side of the obturator foramen 400b, whereas its proximal-side portion is protruding from the body surface 600 on the side of the obturator foramen 400a.
[8] Next, that portion of the medical tube assembly 1 which is protruding from the body surface 600 on the side of the obturator foramen 400b is grasped with one hand, and, keeping this condition, a proximal portion of the guide wire 40 is grasped with the other hand and pulled proximally. This results in that, as shown in FIG. 8, the guide wire 40 is pulled out of the medical tube assembly 1 (medical tube 2), whereas the medical tube assembly 1 is left extending through the puncture-through hole 500.
[9] Subsequently, the head part 24 of the medical tube 2 is grasped with one hand, the flange portion 232 is grasped with the other hand, and the head part 24 and the flange portion 232 are pulled in opposite directions, as shown in FIG. 9. This causes the medical tube 2 to be separated at the separation part 235 into the first tube 233 and the second tube 234.
[10] Next, the first tube 233 and the second tube 234 are respectively drawn out of the puncture-through hole 500, resulting in that the implant 8 is left extending through the puncture-through hole 500, as shown in FIG. 10. The implant 8 is in a state where its distal-side portion is protruding from the body surface 600 on the side of the obturator foramen 400b, whereas its proximal-side portion is protruding from the body surface 600 on the side of the obturator foramen 400a. Then, the distal-side portion and the proximal-side portion of the implant 8 are pulled with predetermined forces, respectively. This generates a tension on the implant 8, whereby the urethra 100 is pulled in a direction for spacing away from the vagina 200, and supported from below by the implant 8.
[11] Subsequently, as shown in FIG. 11, unnecessary portions of the implant 8 are cut away, and predetermined wound closure and the like are carried out, to complete the procedure.

In such a procedure as above, namely, in treatment of urinary incontinence, at the time of inserting the implant 8 into the puncture-through hole 500 so as to set the implant 8 indwelling there, the implant 8 is preliminarily housed in the medical tube 2 and is inserted into the puncture-through hole 500 together with the medical tube 2. Since the medical tube 2 is rigid at least at its curved section 231 as aforementioned, the medical tube 2 is prevented, even within the puncture-through hole 500, from being unwillingly deformed through being crushed by the biological tissue 700. Accordingly, the operation for insertion into the puncture-through hole 500 can be carried out easily and reliably.

In addition, in this procedure, after the implant 8 is inserted into the puncture-through hole 500 together with the medical tube 2, it is possible to separate the medical tube 2 and draw the medical tube 2 out of the puncture-through hole 500 easily. This ensures that the implant 8 can be reliably placed to indwell in the puncture-through hole 500.

Besides, in this procedure, the operation [8], or the operation of pulling the guide wire 40 out, may be omitted.

### <Second Embodiment>

FIG. 16 is a cross-sectional view showing a second embodiment of the medical tube (medical tube assembly) of the present invention.

Hereafter, referring to this drawing, the second embodiment of the medical tube and the medical tube assembly according to the present invention will be described. The following description will center on differences from the aforementioned embodiment, and descriptions of the same items as above will be omitted.

This embodiment is the same as the first embodiment above, except for a difference in the number of lumens formed.

As shown in FIG. 16, a medical tube 2A in this embodiment is formed therein with a lumen 26 in which to insert the guide wire 40, in addition to a lumen 25 in which to insert the implant 8. The lumen 25 and the lumen 26 are formed independently from each other. This prevents interference between the implant 8 and the guide wire 40. Accordingly, it is possible, for example, to smoothly perform insertion and pulling-out of the guide wire 40.

Note that while the number of the lumens formed is two in this embodiment, this is not limitative; for example, the number may be three or more.

### <Third Embodiment>

FIGS. 17 to 19 are sectional views for sequentially illustrating a method of using the medical tube (medical tube assembly) of the present invention in a third embodiment thereof.

Hereafter, referring to these figures, the third embodiment of the medical tube and the medical tube assembly according to the present invention will be described. The following description will center on differences from the aforementioned embodiments, and descriptions of the same items as above will be omitted.

This embodiment is the same as the first embodiment above, except that the separation part is omitted from the medical tube.

As shown in FIGS. 17 and 18, a medical tube 2B in this embodiment has a configuration in which such a separation part 235 as possessed by the medical tube 2 in the first embodiment above is omitted.

In addition, a distal portion 81 of the implant 8 is preliminarily set protruding from a distal end opening 21 of the medical tube 2B.

An example of the method of using the medical tube assembly 1 having the medical tube 2B configured as above will be described, referring to FIGS. 17 to 19. This using method and the using method in the first embodiment above are the same in regard of the operations [1] to [6] and the operations [10] and [11], and are different in regard of the operations [7] to [9]. Here, the different operations will be described.

[7'] After the operation [6], the medical tube assembly 1 in a state where the implant 8 is inserted in the medical tube 2B is prepared. As aforementioned, the distal portion 81 of the implant 8 is protruding from the distal end opening 21 of the medical tube 2B.

Then, in a state where a proximal portion of the guide wire 40 is inserted in the distal end opening 21 of the medical tube 2B, the medical tube assembly 1 is pushed distally relative to the guide wire 40, as shown in FIG. 17. This causes the medical tube assembly 1 to be inserted into and passed through the puncture-through hole 500, and brought into a state where its distal-side portion is protruding from the body surface 600 on the side of the obturator foramen 400b, whereas its proximal-side portion is protruding from the body surface 600 on the side of the obturator foramen 400a. Even in this state, the distal portion 81 of the implant 8 is protruding from the distal end opening 21 of the medical tube 2B.

[8'] Next, that portion of the medical tube assembly 1 which is protruding from the body surface 600 on the side of the obturator foramen 400b is grasped with one hand, and, keeping this condition, a proximal portion of the guide wire 40 is grasped with the other hand and pulled proximally. This results in that, as shown in FIG. 18, the guide wire 40 is drawn out of the medical tube assembly 1 (medical tube 2B), whereas the medical tube assembly 1 is left extending through the puncture-through hole 500. Note that, even in this state, the distal portion 81 of the implant 8 is protruding from the distal end opening 21 of the medical tube 2B.

[9'] Subsequently, the distal portion 81 of the implant 8 is grasped with one hand, and, keeping this condition, a flange portion 232 of the medical tube 2B is grasped with the other hand and pulled proximally until the medical tube 2B is completely drawn out of the puncture-through hole 500. This results in that the implant 8 is left extending through the puncture-through hole 500.

Thereafter, the operations [10] and [11] are carried out sequentially.

Note that while the distal portion 81 of the implant 8 is preliminarily set protruding from the distal end opening 21 in the medical tube 2B, this is not restrictive, and the distal portion 81 may be set recessed to the depth side from the distal end opening 21. In this case, it is preferable, for example, for the medical tube 2B to be so configured that the tube main body 23 and the head part 24 are separable from each other. This ensures that when the head part 24 is separated, the distal portion 81 of the implant 8 is let protrude from the tube main body 23. With the head part 24 separated after the operation [8'], the operation [9'] can be carried out reliably.

While the medical tube and the medical tube assembly of the present invention have been described hereinabove with reference to the embodiments shown in the drawings, the invention is not limited to the embodiments. Each of the components of the medical tube and the medical tube assembly can be replaced with one having an arbitrary configuration that is able to exhibit an equivalent function. Besides, an arbitrarily configured body or bodies may be added.

In addition, the medical tube and the medical tube assembly of the present invention may each be a combination of arbitrary two or more configurations (features) of the embodiments above.

Besides, while the curved section of the medical tube is so configured as to maintain the curved state in the circular arc shape by its own rigidity in each of the embodiments above, this configuration is not restrictive. For example, a configuration may be adopted wherein a rigid stylet having a curved portion curved in a circular arc shape or the like member is inserted in the curved section of the medical tube, so as to maintain the curved state.

In addition, the separation part of the medical tube may be composed of perforations.

### Industrial Applicability

The medical tube of the present invention is a medical tube in which an elongated implant is to be inserted, wherein the medical tube includes a tube having a distal end opening where a distal end thereof is opening, and a proximal end opening where a proximal end thereof is opening, and the tube has a curved section where an intermediate portion in a longitudinal direction thereof is curved and where a curved state is maintained. Therefore, when the medical tube is inserted into a living body, the curved section is prevented from being crushed (compressed) inside the living body. This ensures that, for example in the case where an implant is preliminarily inserted in the medical tube, the operation of inserting the medical tube into a living body together with the implant can be carried out easily and reliably. In addition, after the inserting operation, it is required only to pull the medical tube out of the living body, whereby the implant is left as it is, so that the implant is easily and reliably placed to indwell in the living body.

Accordingly, the medical tube of the present invention has industrial applicability.

### Description of Reference Symbols

1: Medical tube assembly
10: Puncture apparatus
2, 2A, 2B: Medical tube (tube)
21: Distal end opening
22: Proximal end opening
23: Tube main body
231: Curved section
232: Flange portion
233: First tube
234: Second tube
235: Separation part
236: Proximal portion
237: Distal portion
238: Gradually decreasing width portion
239: Stepped part
24: Head part
241: Enlarged diameter portion
242, 243: Tapered portion
25, 26: Lumen
27: Marker
3: Puncture member
31: Puncture needle
315: Needle tip
316: Tapered portion
32: Interlock portion
33: Shaft portion
331, 332: Flange
34: Grasping unit
8: Implant
81: Distal portion
20: Support member
30: Outer tube
301: Distal end opening
302: Proximal end opening
303: Tapered portion
304: Flange portion
40: Guide wire
100: Urethra
200: Vagina
300: Pelvis
400a, 400b: Obturator foramen
500: Puncture-through hole (puncture hole)
600: Body surface
700: Biological tissue
O: Center-of-curvature
ϕd: Outside diameter
w₀, w₁, w₂: Width

## Claims

1. A medical tube in which an elongated implant is to be inserted, comprising
a tube having a distal end opening where a distal end thereof is opening, and a proximal end opening where a proximal end thereof is opening, the tube having a curved section where an intermediate portion in a longitudinal direction thereof is curved and where a curved state is maintained.

2. The medical tube according to claim 1, wherein the tube is rigid at least at the curved section thereof.

3. The medical tube according to claim 1 or 2, wherein the curved section is curved in a circular arc shape.

4. The medical tube according to any of claims 1 to 3, comprising
a separation part where the tube is separable at an intermediate portion in the longitudinal direction thereof.

5. The medical tube according to claim 4, wherein the separation part is disposed at a central portion in a longitudinal direction of the curved section.

6. The medical tube according to claim 5, wherein the tube is provided with a marker for grasping of the central portion in the longitudinal direction of the curved section.

7. The medical tube according to any of claims 4 to 6,
wherein the tube is separated at the separation part into a first tube on a distal side and a second tube on a proximal side; and
the separation part is a part having a fitting structure in which a distal portion of the second tube is fitted in a proximal portion of the first tube, before separation of the tube into the first tube and the second tube.

8. The medical tube according to any of claims 1 to 7, wherein the medical tube is circular in cross-sectional shape at least near the distal end opening.

9. The medical tube according to claim 8, wherein an enlarged diameter portion where the medical tube is enlarged in outside diameter is provided near the distal end opening.

10. The medical tube according to claim 8 or 9, wherein the medical tube is flat in cross-sectional shape at its portion proximally of its portion near the distal end opening.

11. The medical tube according to claim 10, wherein the width of the flat shape is equal to or greater than a maximum outside diameter near the distal end opening of the medical tube.

12. The medical tube according to claim 10 or 11, wherein a thickness direction of the flat shape is oriented toward a center-of-curvature of the curved section.

13. The medical tube according to any of claims 1 to 12, comprising
at least one lumen which opens respectively at the distal end opening and at the proximal end opening,
wherein the implant is inserted in the lumen.

14. The medical tube according to claim 13, comprising
a plurality of the lumens,
wherein a guide wire is inserted in each of other lumen or lumens than that one of the lumens in which the implant is inserted.

15. A medical tube assembly comprising:
the medical tube according to any of claims 1 to 14; and
an elongated implant which is inserted in the medical tube.
